# EUROPEAN PATENT APPLICATION

(11) **EP 1 084 624 A2**
(43) Date of publication of application: **21.03.2001**
(21) Application number: 00308132.0
(22) Date of filing: 18.09.2000
(51) Int. Cl.: A23K 3/00, C12R 1/225

(54) **Ensilage**

(30) Priority: 17.09.1999 GB 9921890
(71) Applicant: Genus Plc, Crewe, Cheshire CW1 6ZY (GB)
(72) Inventor: Davies, David of Bwich y Blaen, Ceredigion, SY25 6DP (GB); Merry, Roger of Perlon, Ceredigion, SY23 5NA (GB); Winters, Ana of 3 Bryncastell, SY24 5DE (GB); Bakwell, Elenor of 2 Gwynfryn, Ceredigion, SY23 4JT (GB)
(74) Representative: Quest, Barry

(57) **Abstract**

Plant material is converted to silage by treatment with new strains of *Lactobacillus plantarum* or *Lactobacillus paracasei subspecies paracasei* having fructan degrading properties.

## Description

This invention relates to the ensilage of plant material.

It is known practice to inoculate plant material with microorganisms in the production of storable silage for cattle feeding.

Lactic acid bacteria (LAB) strains are used as silage inoculants. Lactic acid is produced which helps preservation and improves digestibility.

Mould formation and aerobic spoilage is a known problem with silage and this can be exacerbated by inoculant bacteria strains.

Also, LAB strains require a substrate energy source for effective fermentation, and this may be limited by the amount and availability of e.g. grass water soluble carbohydrate.

An object of the present invention is to provide an effective ensilage inoculant.

In accordance with the invention surprising benefits can be obtained with certain *Lactobacillus* organisms which have been found to have fructan degrading properties.

According to one aspect of the invention therefore there is provided a method of ensilage of plant material in which the plant material is treated with *Lactobacillus* inoculant, characterised in that the inoculant comprises at least one *Lactobacillus* strain having fructan degrading properties.

The invention also provides an inoculant for use in ensilage comprising at least one *Lactobacillus* strain having fructan degrading properties.

Fructans are polysaccharide fructose residues which are naturally present in plant material.

*Lactobacillus* strains do not in general degrade fructans. The present invention is based on the discovery and realisation that there are *Lactobacillus* strains which do have this property.

This means that the fructan content of the plant material can be used as an energy source for the microorganisms. Fermentation can be readily attained leading to effective ensilage with good control of aerobic spoilage.

Since fructans account for up to 80% of the total sugar content of grasses, the ability to degrade fructans constitutes a significant advantage of these microorganisms over non-fructose-degrading bacteria. The fructan-degrading *Lactobacillus* strains can utilise between two and five times the amount of available plant sugars as *Lactobacillus* strains that cannot degrade fructans, and can work at double the rate (e.g. pH can be dropped to the required level within 2 days compared with 4 days for other known inoculants).

The fructan-degrading *Lactobacillus* strain may be a *Lactobacillus plantarum* strain. Thus, the strain may be the *Lactobacillus plantarum* strain as deposited under the Budapest Treaty in the culture collection of the National Collections of Industrial Food and Marine Bacteria Ltd (NCIMBI, 23 St. Machar Drive, Aberdeen, AB24 3RY on 10 September 1999 under Accession Number NCIMB 41028.

This strain was isolated from perennial rye grass and it has had the ability to ferment inulin (a fructan) and also has weak activity against starch.

The invention therefore also provides a microorganism of species *Lactobacillus plantarum,* deposited in the NCIMB culture collection under Accession Number NCIMB 41028, or a mutant or variant thereof having the property of degrading fructans.

Alternatively, the *Lactobacillus* strain may be a *Lactobacillus paracasei subspecies paracasei* strain. Thus, the strain may be *Lacto.para.paracasei* strain deposited under the Budapest Treaty in the NCIMB culture collection on 10 September 1999 under Accession number NCIMB 41029.

The invention therefore also provides a microorganism of species *Lactobacillus paracasei subspecies paracasei*, deposited in the NCIMB culture collection under Accession number NCIMB 41029, or a mutant or variant thereof having the property of degrading fructans.

The plant material utilised in accordance with the invention may be a forage or cereal crop or any other suitable material.

In accordance with the invention, fructans are utilised as an energy source whereby the invention is particularly applicable to difficult ensiling conditions or where the water soluble carbohydrate content of the grass or other plant material is limiting.

The invention has benefits in relation to the attainment of desired silage fermentation characteristics. Also, of considerable possible benefit is the reduction of degradation of forage true protein during ensilage of low sugar grasses.

### Example

In accordance with one example of the invention, for use with grass, maize and wholecrop silage, a microorganism, as described above may be supplied in a vial with a test kit and a suitable growth medium.

A 25 litre plastic drum is filled with hot/cold water to about 30-33°C (not below 30°C and not above 35°C). The growth medium (supplied in a plastic bag) is added to the drum which is shaken gently to dissolve.

The contents of the bacterial vial are then added and the drum is shaken to dissolve the bacterial pellet/powder. The drum is then placed on a heater unit under an insulating cover and the mixture is incubated for 14-16 hours.

The mixture is tested with pH indicator paper until a pH of 4.6 or below is obtained. Bacterial numbers increase until pH 4.2. Below pH 4.2 bacterial multiplication ceases. If the pH is 4.9 or above incubation is continued until the required pH is reached.

On reaching the required pH the drum can be removed from the heater and kept cool until required for use.

When required for use, the contents of the drum can be diluted as appropriate. For example, in the case of 0.85 litres per tonne, 52 litres of product is added to 149 litres of water and applied evenly to 240 tonnes of forage. In the case of 1.7 litres per tonne, 26 litres of product is added to 175 litres of water and applied evenly to 120 tonnes of fresh forage.

A suitable applicator is used and, after each cut of silage, the applicator is flushed with clean water followed by hypochlorite solution (50ppm available chlorine) and then flushed with clean water again.

With this procedure, inoculant of the invention improves the natural lactic acid fermentation of the silage, and the microorganism used has important fructan degrading properties.

Experiments have been performed that demonstrate the beneficial effects of treatment of silage with inoculant of the invention.

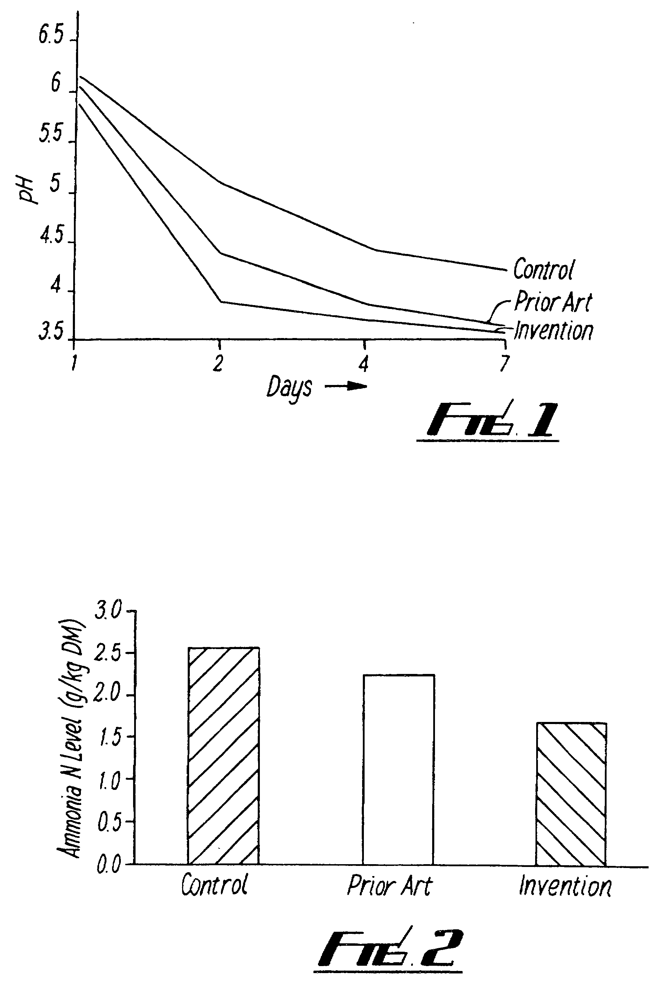

Figure 1 shows data from a representative experiment in which the decrease in pH of silage, a result of fermentation, was measured over a period of 7 days. Following treatment of the silage with an inoculant of the invention (*Lactabacillus plantarum*) as hereinbefore described, the silage pH fell to a predetermined target level after approximately 2 days. Following treatment of the silage with a prior art inoculant, this target pH was only reached after 4 days. In the absence of treatment of the silage (control), the target pH was not attained after 7 days.

Silage quality can be measured in terms of ammonia content, since silage of high quality produces less ammonia than poorer quality silage. This relatively low ammonia content makes the high quality silage more palatable to cattle.

Figure 2 contains data from a representative experiment in which the level of ammonia in silage was measured under various conditions. Treatment of silage with an inoculant of the invention (*Lactobacillus plantarum*) as hereinbefore described significantly reduced the level of ammonia relative to untreated (control) silage. Prior art inoculant was also found to reduce the ammonia level, but was considerably less effective than the inventive inoculant.

Preliminary results obtained from feeding trials demonstrate a significant (28%) increase in the rate of growth of cattle fed with silage treated with an inoculant of the invention (*Lactobacillus plantarum*) relative to cattle fed with untreated silage or silage treated with a prior art inoculant.

It is to be understood that the invention is not intended to be restricted to the details of the above Example.

## Claims

1. A method of ensilage of plant material in which the plant material is treated with *Lactobacillus* inoculant, characterised in that the inoculant comprises at least one *Lactobacillus* strain having fructan degrading properties.

2. A method according to claim 1 characterised in that the *Lactobacillus* strain is a *Lactobacillus plantarum* strain.

3. A method according to claim 2 characterised in that the strain is that deposited under Accession number NCIMB 41028.

4. A method according to claim 1 characterised in that the *Lactobacillus* strain is a *Lactobacillus paracasei subspecies paracasei* strain.

5. A method according to claim 4 characterised in that the strain is that deposited under Accession number NCIMB 41029.

6. A method according to any one of claims 1 to 5 characterised in that the plant material is a forage or cereal crop.

7. A method according to any one of claims 1 to 6 comprising the steps of adding the inoculant in pellet/powder form to a drum containing water and growth medium, dissolving the inoculant, heating the drum to incubate the inoculant/growth medium/water mixture, monitoring the pH of the mixture, cooling the drum after reaching a predetermined pH value, diluting the contents of the drum, and applying the diluted contents to forage.

8. A method according to claim 7 characterised in that the predetermined pH value is 4.9.

9. An inoculant for use in ensilage comprising at least one *Lactobacillus* strain having fructan degrading properties.

10. An inoculant according to claim 9 characterised in that the strain is that stated in any one of claims 2-5.

11. A kit for use in ensilage comprising a vial containing an inoculant according to claim 8 or 9, a test kit and a growth medium.

12. A microorganism of species *Lactobacillus plantarum*, deposited in the NCIMB culture collection under Accession Number NCIMB 41028, or a mutant or variant thereof having the property of degrading fructans.

13. A microorganism of species *Lactobacillus paracasei subspecies paracasei*, deposited in the NCIMB culture collection under Accession number NCIMB 41029, or a mutant or variant thereof having the property of degrading fructans.

14. Ensilage when made by the method of any one of claims 1 to 8.
